**Europäisches Patentamt**

**European Patent Office**

**Office européen des brevets**

(19)

(11) Veröffentlichungsnummer : **0 277 199 B1**

(12) # EUROPÄISCHE PATENTSCHRIFT

(45) Veröffentlichungstag der Patentschrift :
**29.01.92 Patentblatt 92/05**

(51) Int. Cl.$^5$ : **A61F 13/08, A61F 5/01**

(21) Anmeldenummer : **87905434.4**

(22) Anmeldetag : **30.07.87**

(86) Internationale Anmeldenummer :
**PCT/EP87/00419**

(87) Internationale Veröffentlichungsnummer :
**WO 88/00819 11.02.88 Gazette 88/04**

(54) **ELASTISCHE KNIEBANDAGE.**

(30) Priorität : **31.07.86 DE 3625952**

(43) Veröffentlichungstag der Anmeldung :
**10.08.88 Patentblatt 88/32**

(45) Bekanntmachung des Hinweises auf die
Patenterteilung :
**29.01.92 Patentblatt 92/05**

(84) Benannte Vertragsstaaten :
**AT BE CH DE FR GB IT LI LU NL SE**

(56) Entgegenhaltungen :
**EP-A- 0 115 029**
**DE-C- 664 711**
**GB-A- 2 019 726**
**US-A- 3 318 305**

(56) Entgegenhaltungen :
**US-A- 4 084 584**
**US-A- 4 116 236**
**US-A- 4 201 203**
**US-A- 4 287 885**

(73) Patentinhaber : **Habermeyer, Peter, Dr.**
**Oberföhringer Strasse 27**
**W-8000 München 81 (DE)**

(72) Erfinder : **Habermeyer, Peter, Dr.**
**Oberföhringer Strasse 27**
**W-8000 München 81 (DE)**

(74) Vertreter : **Dipl.-Phys.Dr. Manitz Dipl.-Ing.,**
**Dipl.-W.-Ing. Finsterwald Dipl.-Ing. Grämkow**
**Dipl.-Chem.Dr. Heyn Dipl.-Phys. Rotermund**
**Morgan, B.Sc.(Phys.) Robert-Koch-Strasse 1**
**W-8000 München 22 (DE)**

EP 0 277 199 B1

## Beschreibung

Die Erfindung betrifft eine elastische Kniebandage in Schlauchform mit einem die Kniescheibe zumindest teilweise umschließenden, elastischen Führungselement, bei der ein zumindest knieinnenseitig gelegener Teil-Umfangsbereich zumindest in Radialrichtung unelastisch ausgebildet und mit dem Führungselement verbunden ist.

Elastische Kniebandagen in Schlauchform aus gestricktem oder gewirktem Gewebe sind bekannt und werden bei Kniebeschwerden als Stützbandage getragen.

Es ist auch bereits bekannt, in derartige Kniebandagen ein die Kniescheibe umgebendes Ringelement aus hochelastischem Material zu integrieren, um die Kniescheibe in der Beugestellung von der dabei auftretenden Druckkraft der Bandage zu entlasten und die entsprechenden Druckkräfte nur auf die unterhalb des Rings liegenden Weichteile weiterzugeben. Bei Bewegung des Kniegelenks wird durch den die Kniescheibe umgebenden Ring eine ständige Massagewirkung erzielt, die zu einer Verbesserung der Durchblutung der Gelenkweichteile, zu einer beschleunigten Resorption von Ödemen und Ablagerungen und zu einer schnelleren Beseitigung von Ergüssen beiträgt.

Aus der US-A 4 287 885 ist eine Kniebandage der eingangs angegebenen Art bekannt, bei der im Bereich der Patella auf der der Haut zugewandten Innenseite der elastischen Schlauchbandage ein in Umfangsrichtung unelastisches, flexibles Material angebracht ist, auf dem wiederum der Haut zugewandt freiliegende, federnd ausgebildete Führungselemente befestigt sind. Das unelastische Material besteht dabei vorzugsweise aus Klettmaterial, an dem die Führungselemente über entsprechende Klettverschlußverbindungen befestigbar sind. Eine sichere Führung der Patella ist durch die nur oberflächlich befestigten Elemente nicht gewährleistet, und außerdem ist eine derartige Bandage beim Gebrauch nicht stabil genug, da die freiliegenden Führungselemente bei längerer Beanspruchung und häufigerem Wechsel der Bandage leicht beschädigt oder abgerissen werden können.

Aufgabe der Erfindung ist es, eine elastische Kniebandage der eingangs angeführten Art unter Aufrechterhaltung der sowohl für das Tragen der Bandage als auch für die Massagefunktion im Kniescheibenbereich erforderlichen Elastizität gleichzeitig so auszubilden, daß Lateralabweichungen der Kniescheibe beim Abwinkeln des Knies verhindert bzw. solchen Lateralabweichungen wirksam entgegengewirkt wird.

Gelöst wird diese Aufgabe nach der Erfindung im wesentlichen dadurch, daß das elastische Führungselement in einer Tasche aus einem vorzugsweise nicht dehnbaren Material angeordnet ist und daß diese Tasche mit dem zumindest in Radialrichtung unelastisch ausgebildeten Teil-Umfangsbereich zweckmäßigerweise an ihrem Innen- und Außenumfang fest verbunden ist.

Dadurch wird erreicht, daß bei der Bewegung des Kniegelenks durch das elastische Führungselement eine ständige Massagewirkung erzielt wird, die zu einer Verbesserung der Durchblutung der Gelenkweichteile, zu einer beschleunigten Resorption von Ödemen und Ablagerungen und zu einer schnelleren Beseitigung von Ergüssen beiträgt, und außerdem wird sichergestellt, daß Lateralabweichungen der Kniescheibe bei einem Beugen des Knies entgegengewirkt wird, um im Falle solcher Lateralabweichungen auftretenden Reibungseffekten wirksam zu begegnen, bzw. diese Reibungseffekte möglichst zu verhindern.

Die Kniebandage nach der Erfindung erzeugt somit eine aktive Rückhaltekraft für die Kniescheibe, d. h. es wird ein Teil der zum Beugen des Knies aufgewandten Kraft nicht nur zum Dehnen der manschettenförmigen Bandage verwendet, sondern gleichzeitig zur Erzeugung einer im Kniescheibenbereich wirksamen Querkraft, welche die angestrebte Fixierung der Patella ermöglicht.

Vorzugsweise erstreckt sich der nichtelastische Teil-Umfangsbereich über etwa 90° des Gesamtumfangs der manschettenförmigen Kniebandage, und zwar im vorderen Bereich etwa bis zur Längsmittelebene des vorzugsweise um die Kniescheibe ringförmig ausgebildeten Führungselementes, wobei im Überlappungsbereich das nur in Längsrichtung, jedoch nicht in Umfangsrichtung dehnbare material in der Weise mit dem Führungselement verbunden ist, daß das Führungselement ebensowenig wie das nichtelastische Material eine Bewegung in Umfangsrichtung durchführen kann.

Das Führungselement besteht bevorzugt aus einem elastischen, kompressiblem Ring, dessen Innenbereich frei oder vom Bandagenmaterial überspannt sein kann.

Nach einer zweckmäßigen Ausgestaltung der Erfindung ist der in das insbesondere doppellagig ausgebildete Schlauch- bzw. Manschettenmaterial eingearbeitete und lagefixierte, die Kniescheibe formschlüssig umschließende Ring im Querschnitt so ausgebildet, daß er an seiner Außenseite im wesentlichen flach und an der zur Kniescheibe hin gerichteten Seite wulstförmig ausgebaucht ist. Diese Formgestaltung erbringt einerseits eine gute Führungsfunktion bezüglich der Kniescheibe und andererseits eine erwünschte Radialelastizität.

Der nichtelastische Teil-Umfangsbereich besteht zweckmäßigerweise aus einem nichtdehnbaren Streifen eines Gewebematerials, wobei dieser Streifen in die schlauchförmige Bandage als Umfangsbestandteil eingearbeitet oder mit ihr zumindest an seinen Randbereichen fest verbunden, d.h. aufgesetzt ist.

Der nichtdehnbare Streifen überdecktzweckmäßigerweise den die Kniescheibe umschließenden Ring zumindest hälftig, und in diesem Bereich ist auch die den Ring aufnehmende Tasche mit dem nichtdehnbaren Streifen an ihrem Innen- und Außenumfang fest verbunden, wobei diese Tasche bevorzugt aus einem nichtdehnbaren Material besteht, so daß ein allseitig elastischer, d.h. sowohl axial kompressibler als auch radial dehnbarer Ring verwendet werden kann und dennoch die angestrebte Rückhaltefunktion der Kniescheibe erzielt wird.

Weitere vorteilhafte Ausgestaltungen der Erfindung sind in Unteransprüchen angegeben.

Die Erfindung wird nachfolgend anhand der Zeichnung beispielsweise beschrieben; in der Zeichnung zeigt:

Fig. 1 eine Vorderansicht eines Beines mit einer Kniebandage nach der Erfindung,

Fig. 2 eine Ansicht des Innenkniebereichs mit der Bandage nach Fig. 1,

Fig. 3 einen schematischen Querschnitt der Anordnung nach Fig. 1 in Höhe der Kniescheibe,

Fig. 4 die Bandage nach Fig. 1 bei abgewinkelten Knie, und

Fig. 5 eine Prinzipsskizze zur Erläuterung der Kniescheiben-Haltefunktion der Bandage nach Fig. 1.

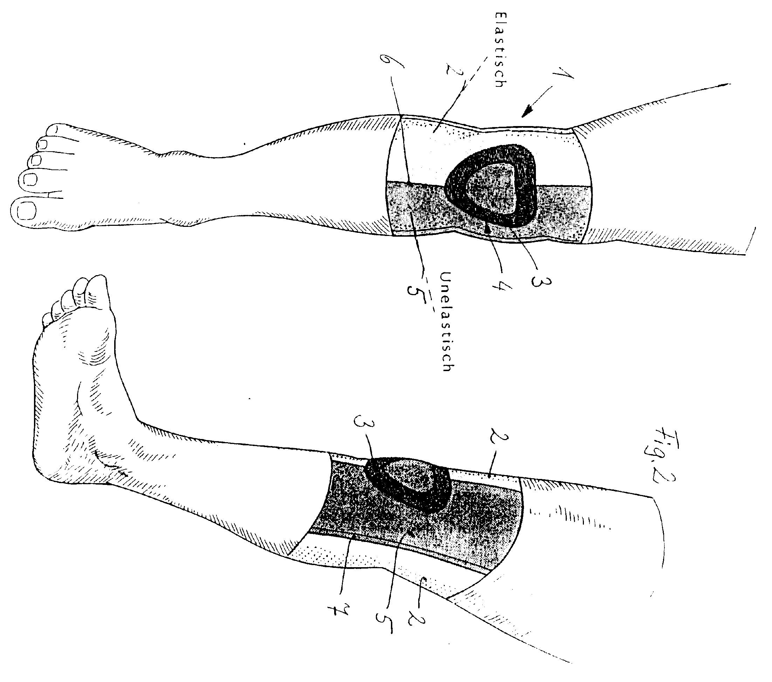

Fig. 1 zeigt eine im Kniebereich angebrachte schlauchförmige Bandage 1, die aus einem vorzugsweise formgestricktem Schlauchteil 2 aus in Umfangsrichtung und in Längsrichtung elastisch dehnbarem Material besteht.

In die Bandage eingearbeitet ist im Bereich der Kniescheibe ein der Umrißform der Kniescheibe etwa angepaßtes, ringförmiges Führungselement 3 aus kompressiblem Material, z.B. weichelastischem Silikon.

Dieses ringförmige Führungselement 3 befindet sich in einer entsprechenden Aufnahmetasche 4. Mit dem elastischen Material 2 fest verbunden und vorzugsweise auf dieses elastische Material aufgesetzt ist auf einem Teil-Umfangsbereich ein Streifen eines in Umfangsrichtung nicht dehnbaren Materials 5. Dieser in Umfangsrichtung unelastische Streifen 5 ist auch mit der das Führungselement 3 aufnehmenden Tasche 4 fest verbunden, d.h. es ist zweckmäßiger Weise der Innenrand und der Außenrand dieser Tasche 4 mit dem Material des Streifens 5 vernäht.

Wenn das Material des ringförmigen Führungselements 3 in Umfangsrichtung dehnbar sein sollte, dann wird als Tasche für diesen Ring 3 ein Material verwendet, das wiederum eine Dehnung in Umfangsrichtung ausschließt, so daß die Tasche selbst und damit auch der sich in ihr befindende Ring 3 keiner Dehnung in Umfangsrichtung ausgesetzt werden kann.

Die Ansicht der Innenknieseite nach Fig. 2 läßt erkennen, daß sich der Streifen 5 aus in Umfangsrichtung nichtdehnbarem Material etwa über einen Umfangswinkelbereich von 90° erstreckt und über seine ganze breite mit dem oberen und dem unteren Rand der Bandage bündig abschließt. Im Bereich des innenknieseitigen Übergangs vom in Umfangsrichtung nichtdehnbaren Material zu dem dehnbaren Material ist vorzugsweise zumindest ein biegsamer Flachstab in die Bandage integriert.

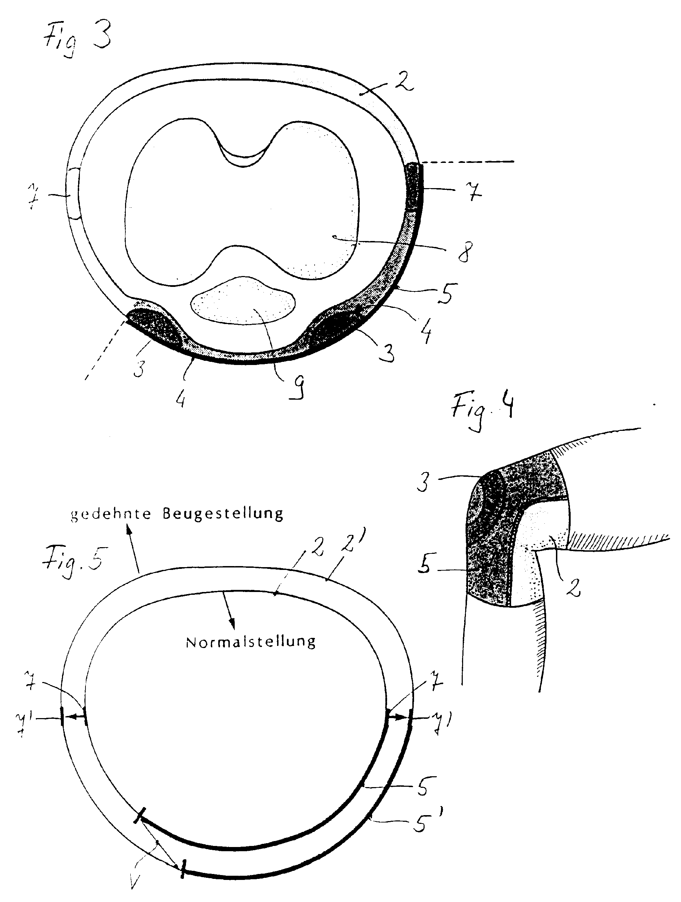

Die schematische Schnittansicht nach Fig. 3 zeigt die das Knie umschließende Bandage 2 aus in Umfangsrichtung und in Längsrichtung elastischem Material mit seitlich integrierten biegsamen Stäbchen 7 und das aufgesetzte und vernähte, in Umfangsrichtung nichtdehnbare Materialteil 5, das mit der ebenfalls in Umfangsrichtung nichtdehnbaren Tasche 4 für das elastische Ringelement 3 fest verbunden ist.

Zu erkennen ist auch die Sollpösition der Kniescheibe 9 bezüglich des Femur kondylus 8 sowie die gewünschte Umschließung der Kniescheibe 9 durch den Führungsring 3.

Die Darstellung nach Fig. 3 entspricht der Normalstellung, d.h. der Stellung bei gestrecktem Knie.

Diese Normalstellung ist in der schematischen Darstellung nach Fig. 5 nochmals gezeigt, d.h. es ist die Position des in Umfangsrichtung nichtdehnbaren Bereichs, der in einer starken Linie ausgezogen ist, relativ zu dem in Umfangsrichtung dehnbaren Bereich der Manschette dargestellt.

Wird das Knie entsprechend der Darstellung in Fig. 4 gebeugt, so tritt eine Durchmesservergrößerung der Manschette ein, wie dies die äußere Darstellung von Fig. 5 zeigt.

Dabei wird deutlich, daß alle in Umfangsrichtung dehnbaren Bereiche der Bandage radial nach außen wandern, wie dies besonders deutlich anhand der seitlich vorgesehenen Flachstäbe 7, 7' zu sehen ist.

Der in Umfangsrichtung nichtdehnbare Bandagenbereich 5 kann aufgrund dieser Undehnbarkeit nicht in entsprechender Weise radial nach außen wandern, sondern er behält bei der sich insgesamt ergebenden Durchmessererhöhung seine Umfangslänge bei, was aber bei der Gesamtbandage zur Folge hat, daß eine Verlagerung des kniescheibenseitigen Endes des nichtdehnbaren Bereichs nach innen erfolgt, wie dies durch den Pfeil V in Fig. 5 angedeutet ist.

Diese Relativverlagerung ist gleichbedeutend mit dem Entstehen einer Zugkraft, die auf die Ringtasche bzw. den Ring 3 wirkt und diesen nach innen zu ziehen versucht bzw. ein seitliches Auswandern des Ringes und damit eine Lateralabweichung der vom Ring gehaltenen Kniescheibe verhindert.

**Patentansprüche**

1. Elastische Kniebandage in Schlauchform mit einem die Kniescheibe zumindest teilweise umschließenden elastischen Führungselement, bei der ein zumindest knieinnenseitig gelegener Teil-Umfangsbereich (5) zumindest in Radialrichtung unelastisch ausgebildet und mit dem Führungselement (3) verbunden ist, dadurch **gekennzeichnet**, daß das elastische Führungselement (3) in einer Tasche (4) aus einem vorzugsweise nicht dehnbaren Material angeordnet ist und daß diese Tasche (4) mit dem zumindest in Radialrichtung unelastisch ausgebildeten Teil-Umfangsbereich (5) zweckmäßigerweise an ihrem Innen- und Außenumfang fest verbunden ist.

2. Elastische Kniebandage nach Anspruch 1, dadurch **gekennzeichnet**, daß sich der unelastische Teil-Umfangsbereich (5) über etwa 90° erstreckt.

3. Elastische Kniebandage nach Anspruch 1 oder 2, dadurch **gekennzeichnet**, daß sich der unelastische Teil-Umfangsbereich (5) etwa bis zur Längsmittelebene (6) des insbesondere zumindest im wesentlichen ringförmig ausgebildeten Führungselementes (3) erstreckt und im Überdeckungsbereich mit dem Führungselement (3) verbunden ist.

4. Elastische Kniebandage nach einem der vorhergehenden Ansprüche, dadurch **gekennzeichnet**, daß das Führungselement (3) aus einem elastisch kompressiblen Ring besteht.

5. Elastische Kniebandage nach Anspruch 4, dadurch **gekennzeichnet**, daß der in das insbesondere doppellagig ausgebildete Schlauchmaterial eingearbeitete und lagefixierte, die Kniescheibe (9) formschlüssig umschließende Ring (3) im Querschnitt wulstförmig und zur Kniescheibe (9) hin ausgebaucht ist.

6. Elastische Kniebandage nach einem der Ansprüche 4 oder 5, dadurch **gekennzeichnet**, daß der Ring (3) zumindest zum Teil aus weichelastischem Material, insbesondere Silikon, besteht.

7. Elastische Kniebandage nach einem oder mehreren der vorhergehenden Ansprüche, dadurch **gekennzeichnet**, daß der unelastische Teil-Umfangsbereich (5) aus einem nichtdehnbaren Streifen eines Gewebematerials besteht, und daß dieser Streifen in die schlauchförmige Bandage als Umfangsbestandteil eingearbeitet oder mit ihr zumindest an seinen Randbereichen fest verbunden ist.

8. Elastische Kniebandage nach einem oder mehreren der vorhergehenden Ansprüche, dadurch **gekennzeichnet**, daß der nichtdehnbare Streifen (5) den Ring (3) hälftig überdeckt.

9. Elastische Kniebandage nach einem oder mehreren der vorhergehenden Ansprüche, dadurch **gekennzeichnet**, daß die den elastischen Ring (3) aufnehmende Tasche (4) aus einem nichtdehnbaren Material besteht.

10. Elastische Kniebandage nach Anspruch 9, dadurch **gekennzeichnet**, daß die gesamte Ringfläche mit nichtdehnbarem Material überspannt ist.

11. Elastische Kniebandage nach einem der vorhergehenden Ansprüche, dadurch **gekennzeichnet**, daß am knieinnenseitigen Längsrand des nichtdehnbaren Streifens (5) am Übergang zum dehnbaren Material (2) zumindest ein biegsamer Flachstab (7) eingearbeitet ist.

12. Elastische Kniebandage nach einem der vorhergehenden Ansprüche, dadurch **gekennzeichnet**, daß der sich an den nicht dehnbaren Streifen (5) anschließende Restumfangsbereich aus einem in Längsrichtung und in Radialrichtung dehnbaren Material besteht.

**Claims**

1. Elastic knee support in hose form, comprising an elastic guide element which at least partly surrounds the knee cap, wherein a partial peripheral region (5) located at least at the inner side of the knee is inelastic at least in radial direction and is connected to the guide element (3), characterised in that the elastic guide element (3) is arranged in a pocket (4) of preferably inextensible material; and in that this pocket is rigidly connected, expediently at its inner and outer periphery, to the partial peripheral region (5) which is inelastic at least in radial direction.

2. Elastic knee support according to claim 1, characterised in that the inelastic partial peripheral region (5) extends over about 90°.

3. Elastic knee support according to claim 1 or claim 2, characterised in that the inelastic partial peripheral region (5) extends to about the longitudinal middle plane (6) of the guide element (3), which is in particular realised at least substantially in the form of a ring, and is connected to the guide element (3) in the superimposed region.

4. Elastic knee support according to one of the preceding claims, characterised in that the guide element (3) consists of an elastic compressible ring.

5. Elastic knee support according to claim 4, characterised in that the ring (3), which surrounds the knee cap (9) in a form-locked manner is bead-shaped in cross-section and bulges towards the knee cap (9) and is integrated and fixed in position in the hose which is in particular formed in two layers.

6. Elastic knee support according to one of the claims 4 or 5, characterised in that the ring (3) is at least partly comprised of a soft-elastic material, in particular of silicone.

7. Elastic knee support according to one of the preceding claims, characterised in that the the inelastic partial peripheral region (5) is comprised of an inextensible strip of fabric material; and in that this strip is integrated into the hose-like support as a peripheral component, or is rigidly connected thereto at least at its edge regions.

8. Elastic knee support according to one of the preceding claims, characterised in that the inextensible strip (5) covers half the ring (3).

9. Elastic knee support according to one of the preceding claims, characterised in that the pocket (4) which accommodates the elastic ring (4) is comprised of an inextensible material.

10. Elastic knee support according to claim 9, characterised in that the entire ring surface is covered with inextensible material.

11. Elastic knee support according to one of the preceding claims, characterised in that at least one pliable flat bar (7) is integrated in the longitudinal edge of the inextensible strip (5) at the inner side of the knee and at the transition to the extensible material (2).

12. Elastic knee support according to one of the preceding claims, characterised in that the remaining peripheral region which follows the inextensible strip (5) is comprised of a material which is extensible in the longitudinal direction and in the radial direction.

**Revendications**

1. Genouillère élastique de forme tubulaire comprenant un élément de guidage élastique qui entoure au moins partiellement la rotule, dans laquelle une zone périphérique partielle (5) disposée au moins du côté intérieur au genou est réalisée de façon non-élastique au moins en direction radiale et est reliée à l'élément de guidage (3), caractérisée en ce que l'élément de guidage élastique (3) est agencé dans une poche (4) en matériau de préférence inextensible, et en ce que cette poche (4) est reliée fermement, de façon appropriée à sa périphérie intérieure et sa périphérie extérieure, à la zone périphérique partielle (5) réalisée de façon non-élastique au moins en direction radiale.

2. Genouillère élastique selon la revendication 1, caractérisée en ce que la zone périphérique partielle non-élastique (5) s'étend sur environ 90°.

3. Genouillère élastique selon l'une des revendications 1 ou 2, caractérisée en ce que la zone périphérique partielle non-élastique (5) s'étend jusqu'au voisinage du plan longitudinal médian (6) de l'élément de guidage (3), lequel est en particulier formé au moins généralement en forme d'anneau, et est reliée à l'élément de guidage (3) dans la zone de recouvrement.

4. Genouillère élastique selon l'une des revendications précédentes, caractérisée en ce que l'élément de guidage (3) consiste en un anneau compressible élastique.

5. Genouillère élastique selon la revendication 4, caractérisée en ce que l'anneau (3), qui entoure la rotule en épousant sa forme et qui est intégré et fixé en position dans le matériau tubulaire en particulier à double couche, a en section la forme d'un bourrelet et est renflé en direction de la rotule (9).

6. Genouillère élastique selon l'une des revendications 4 ou 5, caractérisée en ce que l'anneau (3) est réalisé au moins en partie en un matériau élastique mou, en particulier en silicone.

7. Genouillère élastique selon l'une des revendications précédentes, caractérisée en ce que la zone périphérique partielle non-élastique (5) est formée par une bande inextensible de matériau textile, et en ce que cette bande est intégrée en tant que composant périphérique de la genouillère tubulaire, ou est reliée à celle-ci au moins dans ses zones de bordure.

8. Genouillère élastique selon l'une des revendications précédentes, caractérisée en ce que la bande inextensible (5) recouvre l'anneau (3) par moitié.

9. Genouillère élastique selon l'une des revendications précédentes, caractérisée en ce que la poche (4) qui reçoit l'anneau élastique (3) est réalisée en un matériau inextensible.

10. Genouillère élastique selon la revendication 9, caractérisée en ce que la totalité de la surface annulaire est recouverte de matériau inextensible.

11. Genouillère élastique selon l'une des revendications précédentes, caractérisée en ce qu'au moins une barrette plane flexible (7) est intégrée du côté intérieur au genou de la bordure longitudinale de la bande inextensible (5) et à la transition vers le matériau extensible.

12. Genouillère élastique selon l'une des revendications précédentes, caractérisée en ce que la zone périphérique restante qui fait suite à la bande inextensible (5) est réalisée en matériau extensible en direction longitudinale et en direction radiale.

Elastisch

Unelastisch

Fig.2

Fig 3

Fig. 4

gedehnte Beugestellung

Fig. 5

Normalstellung